(19) Europäisches Patentamt
European Patent Office
Office européen des brevets

(11) Veröffentlichungsnummer: **0 144 883**
**B1**

(12) **EUROPÄISCHE PATENTSCHRIFT**

(45) Veröffentlichungstag der Patentschrift: **04.02.87**

(51) Int. Cl.⁴: **C 07 C 102/00,** C 07 C 103/133, C 07 C 131/00

(21) Anmeldenummer: **84114256.5**

(22) Anmeldetag: **26.11.84**

(54) **Verfahren zur Herstellung von alpha-substituierten Acrylsäureamiden.**

(30) Priorität: **02.12.83 DE 3343674**

(43) Veröffentlichungstag der Anmeldung: **19.06.85 Patentblatt 85/25**

(45) Bekanntmachung des Hinweises auf die Patenterteilung: **04.02.87 Patentblatt 87/6**

(84) Benannte Vertragsstaaten: **BE CH DE FR GB IT LI NL**

(56) Entgegenhaltungen:
**DE-A-1 044 795**
**DE-A-1 081 884**
**US-A-3 105 741**

**CHEMICAL ABSTRACTS, Band 87, Nr. 17, 24. Oktober 1977, Seite 662, Nr. 134375f, Columbus, Ohio, US; V.I. BOEV et al.: "Use of trichloroacetonitrile as a catalyst of the Beckmann rearrangement"**

(73) Patentinhaber: **BASF Aktiengesellschaft, Carl-Bosch- Strasse 38, D-6700 Ludwigshafen (DE)**

(72) Erfinder: **Merger, Franz, Dr. Chem., Max- Slevogt-Strasse 25, D-6710 Frankenthal (DE)**
Erfinder: **Schwarz, Wolfgang, Dr. Chem., Wesostrasse 132, D-7507 Pfinztal (DE)**

**EP 0 144 883 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein neues Verfahren zur Herstellung von α-substituierten acrylsäureamiden, ausgehend von Oximen α-substituierter Acroleine.

α-substituierte Acrylamide können durch Amidierung der jeweiligen Ester mit Ammoniak oder durch Hydratisierung der entsprechenden Nitrile erhalten werden. Die Synthese der dazu benötigten Acrylester und Acrylnitrile ist aber nur begrenzt möglich und im wesentlichen auf die (Meth)acrylverbindungen beschränkt. Darüber hinaus ist die Amidierung technisch aufwendig und läuft nicht besonders selektiv ab, da Nebenreaktionen, wie Michael-Additionen auftreten können.

Nach dem Vorschlag der US-A 4 365 092 soll die Herstellung von Methacrylamid aus Methacrylsäuremethylester mit wäßriger Ammoniaklösung erfolgen. Hierbei sind jedoch lange Reaktionszeiten erforderlich und man benötigt relativ hohe Mengen teurer anionischer Tenside, welche die Abtrennung und Reinigung des Amids erschweren.

Ein anderer Syntheseweg für Methacrylamid verläuft ausgehend von Acetoncyanhydrin über das Zwischenprodukt Methacrylamid-sulfat. Bei dieser Verfahrensweise fallen jedoch große Mengen an Salzen an, zudem müssen aufwendige Reinigungsschritte durchgeführt werden.

Zur Herstellung von Säureamiden sind aus der Literatur auch Umlagerungen von Aldoximen bekannt (j.Am.Chem.Soc. 83, 1983 (1961); Rec.Trav.Chim. Pays Bas 95, 123 (1976) und 96, 142 (1977)). Die den Aldoximen zugrunde liegenden Aldehyde gehören zumeist der Klasse der gesättigten oder der aromatischen Aldehyde an. Als Katalysatoren dienen Salze von Nickel, Zink, Palladium, Kobalt oder Kupfer. Besonders wirksam erwiesen sich dabei Nickel(II)acetat und Palladium(II)acetat, während dagegen Kupfer(II)acetat bei Benzaldoxim nur Umsetzungen in unerwünschter Richtung oder mit schlechten Ausbeuten liefert.

In der JP-A-128 502/1977 wird neben der Umlagerung von gesättigten Aldoximen und Benzaldoximen auch die Umsetzung von Zimtaldehydoxim in Gegenwart von Kupferacetylacetonat beschrieben. Es hat sich aber gezeigt, daß die Anwendung dieses Komplexsalzes bei der Herstellung von α-alkylsubstituierten Acrylsäureamiden zu keinen befriedigenden Ergebnissen führt.

Schließlich wird in der DE-A-3 205 946 die Herstellung von Methacrylamid, ausgehend von Methacroleinoxim in Gegenwart eines Katalysators, auf Basis Hupter/Chrom, vorgeschlagen. Für diesen Katalysator, der zweckmäßig in Form eines Trägerkatalysators eingesetzt werden soll, ist ein definiertes Molverhältnis von Kupfer zu Chrom erforderlich.

Bei diesem Verfahren liegt die Ausbeute an Methacrylamid aber lediglich bei 72 %. Zudem hat sich gezeigt, daß die alleinige Verwendung der dort genannten Kupfersalze, die den Kupferanteil des Katalysators ausmachen und die sich von den Carbonsäuren Ameisensäure, Essigsäure und Weinsäure ableiten, durchweg nicht die gewünschten optimalen Resultate liefert.

In Anbetracht der bisher bekannt gewordenen unbefriedigenden und meistens auf Methacrylamid beschränkten Synthesen von α-alkylsubstituierten Acrylsäureamiden lag der Erfindung die Aufgabe zugrunde, solche Verbindungen auf einfachere und wirtschaftlichere Weise herzustellen.

Demgemäß wurde gefunden, daß man α-substituierte Acrylsäureamide der allgemeinen Formel

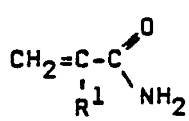

(I),

in der $R^1$ einen geradkettigen, verzweigten oder cyclischen alkylrest mit bis zu 15 Kohlenstoffatomen bedeutet, der gegebenenfalls weiter substituiert sein kann, aus den Aldoximen der allgemeinen Formel

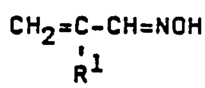

(II),

in der $R^1$ die obengenannte Bedeutung besitzt, vorteilhaft erhält, wenn man die Aldoxime in Gegenwart eines trägerfreien Kupfer(II)carboxylats einer Monocarbonsäure mit 2 bis 18 Kohlenstoffatomen und in Gegenwart eines Nitrils der allgemeinen Formel III

$$CH_2 = \underset{R^1}{\overset{|}{C}} - CN \qquad (III),$$

in der $R^1$ die obige Bedeutung besitzt, als Cokatalysator auf eine Temperatur von 40 bis 250° C erwärmt.

Das erfindungsgemäße Verfahren wird mit Oximen von Acroleinen durchgeführt, die in α-Stellung einen geradkettigen, verzweigten oder cyclischen Alkylrest mit bis zu 15 Kohlenstoffatomen tragen. Dieser Alkylrest kann durch weitere Gruppen, die sich unter den Reaktionsbedingungen indifferent verhalten, substituiert sein, beispielsweise niedere alkoxygruppen (auch in geminaler Stellung), niedere Acyloxygruppen, niedere alkoxycarbonylgruppen oder niedere Mono- und Dialkylaminogruppen.

Beispielhaft seien folgende α-alkylsubstituierten Acroleinoxime erwähnt: α-Methacroleinoxim, α-Ethylacroleinoxim, α-Butylacroleinoxim, α-(2-Ethyl-hexyl)acroleinoxim, α-Nonylacroleinoxim, α-Cyclohexylacroleinoxim, α-(4-Methylcyclohexyl)acroleinoxim, α-(5-Carbethoxypropyl)acroleinoxim oder α-(4,4-Dimethylaminobutyl)acroleinoxim.

Die den Aldoximen (II) zugrundeliegenden α-substituierten acroleine sind mittels des in der EP-A-58 927 beschriebenen Verfahrens durch Umsetzung von Alkanalen mit Formaldehyd und sekundärem Amin in Gegenwart von Säure gut zugänglich.

Die für das erfindungsgemäße Verfahren benötigten Aldoxime lassen sich nach an sich bekannten Methoden, wie sie beispielsweise in Houben-Weyl "Methoden der oganischen Chemie", Band 10/4, S. 55f. beschrieben sind, aus den α-substituierten Acroleinen und Hydroxylammoniumsalzen herstellen, wobei die freiwerdende Säure mit Base neutralisiert wird.

Bei der Herstellung der niederen Homologen dieser Aldoxime, bei denen der gegebenenfalls substituierte α-Alkylrest bis zu 4 Kohlenstoffatome aufweist, kann auf den üblichen Zusatz von Basen verzichtet werden. Die Bildung und Abtrennung der Aldoxime erfolgt ohne Neutralisation der Säure und entsprechende Salzbildung. Die wäßrige Säure kann rückgeführt oder für Neutralisationszwecke verwendet werden.

Vorteilhaft lassen sich technische wäßrige Hydroxylammoniumsalzlösungen einsetzen. Überschüssige Säure und deren Ammoniumsalze, wie sie in solchen technischen Lösungen vorliegen, stören dabei nicht.

Die Umlagerung der Aldoxime zu den entsprechenden α-substituierten Acrylsäureamiden erfolgt bei einer Temperatur von 40 bis 250° C, vorzugsweise 60 bis 180° C und insbesondere 80 bis 150° C.

Vorzugsweise führt man die Umsetzung bei atmosphärischem Druck durch. In manchen Fällen ist es auch vorteilhaft sie unter erhöhtem Druck (bis zu 20 bar) vorzunehmen.

Man kann lösungsmittelfrei oder aber vorzugsweise in Gegenwart eines inerten Lösungsmittels wie Toluol, Xylol, Mesitylen, Chlorbenzol, Nitrobenzol, Tetralin, Decalin, Dioxan, Dibutylether oder Essigsäure-n-butylester arbeiten.

Erfindungsgemäß wird die Umlagerung der Aldoxime in Gegenwart eines trägerfreien Kupfer(II)carboxylats einer Monocarbonsäure mit 2 bis 18 Kohlenstoffatomen vorgenommen.

Als solche Säuren kommen (gegebenenfalls substituierte) Monocarbonsäuren mit einem gesättigten oder ungesättigten geradkettigen, verzweigten oder cyclischen Alkylrest, Aralkansäuren, Oxaalkansäuren sowie aromatische Carbonsäuren in Betracht, beispielsweise Essigsäure, Propionsäure, 2-Chlorpropionsäure, Isobuttersäure, 2-Methylbutansäure, Pentansäure, Hexansäure, 2-Ethylhexansäure, 3,5,5-Trimethylhexansäure, Decansäure, 9-Decensäure, 9-Dodecensäure, 9 Octadecensäure, Cyclohexancarbonsäure, Phenylessigsäure, 1-Phenylcyclopentan-1-carbonsäure, Methoxyessigsäure, Benzoesäure, 3,5-Dichlorbenzoesäure oder Naphthoesäure.

Die Herstellung der für das erfindungsgemäße Verfahren benötigten Kupfersalze kann nach an sich bekannten Methoden, z.B. durch Umsetzung der Carbonsäuren mit Kupfer(II)carbonat oder durch Umsetzung von Kupfersalzen mit den alkali- oder Ammoniumsalzen der jeweiligen Carbonsäure, erfolgen.

Die Kupfersalze werden trägerfrei eingesetzt, d.h. ohne Verwendung eines in der Katalysatortechnik häufig üblichen Trägermaterials und ihre Verwendung erfolgt zweckmäßig in Abwesenheit von Chrom.

Die Menge des eingesetzten Kupfer(II)carboxylats beträgt 0,1 bis 5 Mol%, vorzugsweise 0,5 bis 2 Mol%, jeweils bezogen auf 1 Mol Aldoxim.

Das erfindungsgemäße Verfahren führt man mit Nitrilen der allgemeinen Formel III

$$CH_2 = \underset{R^1}{\overset{|}{C}} - CN \qquad (III),$$

in der $R^1$ die obengenannte Bedeutung besitzt, als Cokatalysatoren durch.

Sie werden in Mengen von 1 bis 200 Mol%, vorzugsweise 20 bis 100 Mol%, jeweils bezogen auf 1 Mol Aldoxim, eingesetzt.

Ein besonderer Vorteil, das erfindungsgemäße Verfahren mit Acrylnitrilen (Formel III) als Cokatalysatoren durchzuführen, liegt darin, daß die Menge des eingesetzten Kupfer(II)carboxylats auf die oben genannten geringen Konzentrationswerte beschränkt werden kann. Daneben beobachtet man den schon von den Umlagerungen der gesättigten bzw. aromatischen Aldoximen (Soc.cit.) gekannten Effekt, daß der Zusatz zu einer Steigerung der Raum-Zeit-Ausbeute führt.

Das erfindungsgemäße Verfahren wird üblicherweise so ausgeführt, daß man das Aldoxim zusammen mit Kupfer(II)carboxylat und dem Nitril, vorzugsweise in einem inerten Lösungsmittel, auf den genannten Temperaturbereich erwärmt. Dabei ist sowohl eine diskontinuierliche als auch eine kontinuierliche Arbeitsweise möglich. Zur besseren Kontrolle der exothermen Reaktion ist es zweckmäßig, nur einen Teil des Oxims vorzulegen und den Rest im Verlauf der Reaktion zuzugeben.

Nach beendigung der Reaktion werden die Zielprodukt durch Kristallisarekt einsetzbar. Sie können durch Umkristallisation gereinigt und gegebenenfalls durch Ionenaustausch von Kupferspuren befreit werden.

Von der als Cokatalysator eingesetzten Nitrilmenge werden nach Ablauf der Reaktion in der Regel durch einfache Destillation 95 bis 100 % zurückerhalten.

Weitere Vorteile des erfindungegemäßen Verfahrens sind die Verwendung der im Vergleich zu den Acrylestern leicht zugänglichen Acroleine (die oft als Vorstufe für die Synthese der acrylester dienen) sowie die unter den Reaktionsbedingungen besonders selektiv ablaufende Aldoximumlagerung.

Die nach dem erfindungsgemäßen Verfahren gewonnenen Amide sind wertvolle Zwischenprodukte für die Herstellung von modifizierbaren Polymeren, z.B. für Dispersionen und Lacke.

Die Erfindung soll durch folgende Beispiele veranschaulicht werden.

Herstellung von Methacroleinoxim

Zu 1510 ml einer Lösung, die 490,3 g Hydroxylammoniumsultat, 19,25 g Schwefelsäure und 30,83 g Ammoniumsulfat enthielt, wurden innerhalb einer Stunde, bei ca. 3°C, unter kräftigem Rühren, 570 g Methacrolein (Reinheit 98%) zugetropft. Dann wurde die Kühlung entfernt und 2 Stunden weitergerührt, wobei die Temperatur des Reaktionsansatzes auf 21°C anstieg. Nach GC-analyse waren 97% des Methacroleins umgesetzt. Man trennte die organische Phase ab, etherte die wäßrige Phase zweimal aus, trocknete die vereinigten organischen Phasen über Magnesiumsulfat und engte am Rotationsverdampfer ein. Der Rückstand wurde über einen Dünnschichtverdampfer (82°C/16 mbar) destilliert. Man erhielt 387 g Methacroleinoxim (Ausbeute 90%, bezogen auf umgesetztes Methacrolein; Reinheit 99,6%).

**Beispiel 1**

30 g Methacroleinoxim, 239,1 g Methacrylnitril und 12,35 g Cu(II)-2-ethyl-hexanoat (= 1 Mol% bezogen auf Oxim) wurden in 1800 g o-Xylol unter Rühren auf 110°C erwärmt. Dann wurden weitere 270 g Methacroleinoxim innerhalb von 15 min zugetropft, nach beendeter Zugabe noch 10 min gerührt und anschließend auf Raumtemperatur abgekühlt. Oas ausgefallene Methacrylamid wurde über eine Druckfilternutsche abfiltriert.

Der Versuch wurde noch dreimal in oben genannter Weise wiederholt, jedoch wurde die Mutterlauge aus dem ersten Ansatz verwendet und kein frisches Methacrylnitril zugesetzt. Insgesamt wurden aus allen vier Ansätzen 1105 g (92,5 %) rohes Methacrylsäureamid erhalten. Beim Aufdestillieren der Mutterlauge wurden 201 g (= 85 %) des eingesetzten Methacrylnitrils zurückerhalten.

**Beispiel 2**

418,8 g α-Nonylacroleinoxim (Reinheit 94 %), 1030 g o-Xylol, 94,6 g α-Nonylacrylnitril (Reinheit 95 %) und 15,9 g Cu(II)-2-ethylhexanoat (2 Mol.%, bezogen auf Oxim) wurden auf 130°C erhitzt. Es setzte eine heftige exotherme Reaktion ein, nach deren Abklingen wurde 1,5 Stunden bei 110°C nachgerührt. Laut GC-Analyse waren 98,8 % des eingesetzten Oxims umgesetzt. Man versetzte das Reaktionsgemisch mit 1,2 1 Petrolether, kühlte auf -20°C ab und saugte das ausgefallene rohe Amid ab. Nach Umkristallisation aus Cyclohexan erhielt man 352 g (84 %)α-Nonylacrylamid als farblose Kristalle, die bei 80 bis 81°C schmolzen.

**Beispiel 3**

In einem 100 1-Rührkessel wurden 35,5 kg Xylol (Isomerengemisch), 1,06 kg Ethylacrylnitril und 0,215 kg Cu(II)-acetat zum Rückfluß (ca. 135°C) erhitzt und anschließend wurden innerhalb von 25 min 7,8 kg Ethylacroleinoxim (Reinheit 83 %) zugegeben. Nach 10 min wurde unter Rühren zunächst mit Wasser, dann mit Solelösung gekühlt, bis die Temperatur des Reaktionsgemisches -6°C erreicht hatte. Das Ethylacrylamid wurde über eine Nutsche abgesaugt und mit Petrolether gewaschen. Die Rohausbeute betrug 5,956 kg (92 %).

**Patentansprüche**

1. Verfahren zur Herstellung von α-substituierten acrylsäureamiden der allgemeinen Formel I

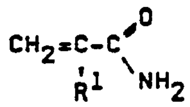 (I),

in der $R^1$ einen geradkettigen, verzweigten oder cyclischen Alkylrest mit bis zu 15 Kohlenstoffatomen bedeutet, der gegebenenfalls weiter substituiert sein kann, aus Aldoximen der allgemeinen Formel II

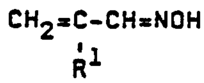 (II),

in der $R^1$ die obengenannte Bedeutung besitzt, dadurch gekennzeichnet, daß man die Aldoxime in Gegenwart eines trägerfreien Kupfer(II)carboxylats einer Monocarbonsäure mit 2 bis 18 Kohlenstoffatomen und in Gegenwart eines Nitrils der allgemeinen Formel III

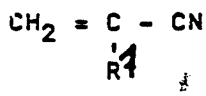 (III),

in der $R^1$ die obige Bedeutung besitzt, als Cokatalysator auf eine Temperatur von 40 bis 250° C erwarmt.

2. Verfahren gemäß Anspruch 1, dadurch gekennzeichnet, daß man die Umsetzung bei einer Temperatur Von 60 bis 180° C durchführt.

3. Verfahren gemäß anspruch 1, dadurch gekennzeichnet, daß man die Umsetzung bei atmosphärischem Druck durchführt.

4. Verfahren gemäß Anspruch 1, dadurch gekennzeichnet, daß man die Umsetzung in Anwesenheit eines inerten Lösungsmittels durchführt.

**Claims**

1. A process for the preparation of an α-substituted acrylamide of the general formula

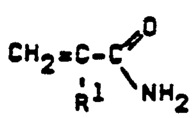

where $R^1$ is a straight-chain, branched or cyclic alkyl radical of up to 15 carbon atoms which can be unsubstituted or further substituted, from an aldoxime of the general formula II.

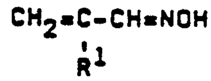

(II),

where $R^1$ has the above meanings, wherein the aldoxime is heated at from 40 to 250°C in the presence of a carrier-free copper(II) carboxylate obtained from a monocarboxylic acid of 2 to 18 carbon atoms and in the presence of a nitrile of the general formula III.

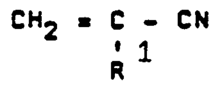

(III),

where $R^1$ has the above meanings, as cocatalyst.

2. A process as claimed in claim 1, wherein the reaction is carried out at from 60 to 180°C.

3. A process as claimed in claim 1, wherein the reaction is carried out under atmospheric pressure.

4. A process as claimed in claim 1, wherein the reaction is carried out in the presence of an inert solvent.

**Revendications**

1. Procédé de préparation d'amides d'acide acrylique alpha-substitué de la formule générale I

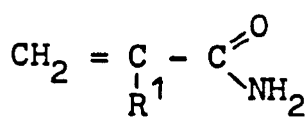

(I),

dans laquelle $R^1$ désigne un groupe alkyle à chaîne droite ou ramifiée ou cyclique, comprenant jusqu'à quinze atomes de carbone et pouvant encore porter des substituants, à partir d'aldoximes de la formule générale II

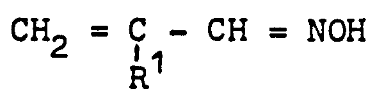

(II),

dans laquelle $R^1$ possède la signification définie, caractérisé en ce que l'on chauffe une telle aldoxime en présence d'un carboxylate cuivrique d'un acide monocarboxylique en $C_2$ à $C_{18}$, non déposé sur un support, et d'un nitrile de la formule générale III

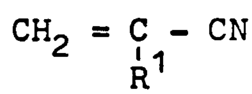

(III),

dans laquelle $R^1$ possède la signification définie, à une température comprise entre 40 et 250°C.

2. Procédé suivant la revendication 1, caractérisé en ce que la réaction est réalisée à une température comprise entre 60 et 180°C.

3. Procédé suivant la revendication 1, caractérisé en ce que la réaction est réalisée à la pression atmosphérique.

4. Procédé suivant la revendication 1, caractérisé en ce que la réaction est effectuée dans un solvant inerte.